# EUROPÄISCHE PATENTSCHRIFT

(11) **EP 1 586 310 B1**
(45) Veröffentlichungstag und Bekanntmachung des Hinweises auf die Patenterteilung: **27.12.2006**
(21) Anmeldenummer: 04101545.4
(22) Anmeldetag: 15.04.2004
(51) Int. Cl.: A61K 9/14, A61K 31/41

(54) **Verfahren zur Herstellung von freifliessenden, pulverförmigen Valsartan-Adsorbaten**
Method to manufacture valsartan adsorbates in the form of a free-flowing powder
Procédé de préparation d'adsorbates de valsartan en forme de poudre coulante

(43) Veröffentlichungstag der Anmeldung: 19.10.2005
(73) Patentinhaber: Helm AG, 20097 Hamburg (DE)
(72) Erfinder: Glänzer, Klaus, 22337, Hamburg (DE)
(74) Vertreter: Becker Kurig Straus

(56) Entgegenhaltungen:
- WO-A-00/04862
- WO-A-03/066606
- WO-A-20/04028505

## Beschreibung

Die vorliegende Erfindung betrifft ein neuartiges Verfahren zur Herstellung von Valsartan-Adsorbaten und/oder von dessen Solvaten oder Hydraten. Die erfindungsgemäßen Adsorbate enthalten in einer besonders bevorzugten Ausführungsform den Wirkstoff oder eines seiner pharmazeutisch akzeptablen Salze und/oder deren Solvate oder Hydrate in fein verteilter amorpher Form. Die Erfindung betrifft weiterhin Valsartan-Adsorbate, die gemäß dem vorgenannten Verfahren herstellbar sind sowie pharmazeutische Formulierungen, zu deren Herstellung die vorgenannten Valsartan-Adsorbate eingesetzt werden. Bevorzugte erfindungsgemäße Arzneiformen sind Tabletten, Kapseln, Pellets und Granulate, die auf an sich bekannte Weise mit üblichen, pharmazeutisch akzeptablen Hilfsstoffen hergestellt werden. Erfindungsgemäß besonders bevorzugt sind den Wirkstoff schnell freisetzende Tabletten, die durch Direktverpressung der erfindungsgemäßen Valsartan-Adsorbate hergestellt werden.

Der unter dem INN Valsartan bekannte Wirkstoff mit der chemischen Bezeichnung (S)-N-(1-carboxy-2-methyl-prop-1-yl)-N-pentanoyl-N-[2'-(1H-tetrazol-5-yl)-biphenyl-4-yl-methylamin, wird durch die folgende Strukturformel dargestellt: 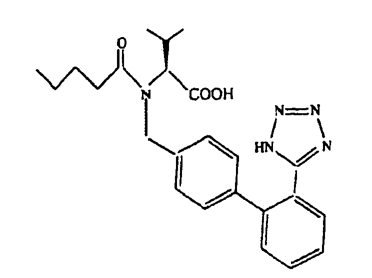 Valsartan gehört zur Klasse der nicht-peptidischen Angiotensin-II-Rezeptor-Antagonisten, mit sehr hoher Selektivität bezüglich der AT₁- Rezeptoren. Mit einer üblichen Tagesdosis von 80 bis 160 mg wird Valsartan als Monopräparat oder in Kombination mit dem Diuretikum Hydrochlorothiazid zur Behandlung kardiovaskulärer Erkrankungen eingesetzt. Der AT₁-Rezeptor-Antagonist Valsartan hemmt insbesondere die Blutdrucksteigerung durch Angiotensin-II, unterdrückt die Angiotensin-II-induzierte Aldosteronsekretion und vermindert die Angiotensin-II-induzierte Flüssigkeitsaufnahme (siehe z. B. Allgemeine und spezielle Pharmakologie und Toxikologie, Hrsg. W. Forth, D. Henschler, W. Rummel, U. Förstermann und K. Starke, 8. Aufl., Urban & Fischer, München/Jena, 2001).

EP 0 443 983 B1, Beispiel 16, beschreibt Valsartan, seine pharmazeutisch akzeptablen Salze und Verfahren zur Herstellung. Die Herstellung von Valsartansalzen wird beispielsweise aber auch in der WO 02/06253 beschrieben.

Aus dem Stand der Technik ist es bekannt, dass Valsartan nicht nur als amorpher Feststoff vorliegt, sondern in mehreren kristallinen Formen, teilkristallinen Formen oder als Mischung verschiedener Polymorphe mit amorphem Material vorliegen kann (vgl. WO 02/06253; WO 03/089417). Die in verschiedenen Patentdokumenten gemachten Angaben zu den Schmelzpunkten von Valsartan-Polymorphen schwanken erheblich, wobei von Werten zwischen 80°C und 117°C berichtet wird. WO 03/089417 beschreibt eine Form I-Valsartan mit Schmelzpunkten zwischen 80°C bis 91°C und Form II-Valsartan zwischen 91°C bis 102°C. Das mittels verschiedener Syntheserouten hergestellte Valsartan, die in EP 0 443 963 B1 angegeben sind, zeigte Schmelzpunkte von 105°C bis 115°C (D); 105°C bis 115°C (C); 116°C bis 117°C (B); 105°C bis 115°C (A), ohne jedoch auf spezifische Polymorphe einzugehen. Im Merck-Index, 13th Edition, wird für Valsartan-Kristalle aus Diisopropylether ein Schmelzpunkt von 116°C bis 117°C angegeben. Es ist daher festzustellen, dass die Herstellung einer definierten polymorphen Form außerordentlich stark von Prozessparametern bzw. Lösungsmittelzusammensetzungen abhängig ist und bei geringfügigen Abänderungen dieser Parameter unterschiedliche Produkte resultieren. Dies hat eine sehr aufwendige Prozesssteuerung und Qualitätskontrolle zur Folge, da ein reproduzierbar herstellbares und sauber definiertes Polymorph sowohl zur Erfüllung regulatorischer Anforderungen als auch zur Sicherung der gleichbleibenden Qualität des Arzneimittels und damit der Gewährleistung der pharmazeutischen Eigenschaften bzw. der Einnahmesicherheit für den Patienten unbedingt erforderlich ist. Eine Möglichkeit dieses Problem zu lösen und zu einem vorteilhafteren Isolationsprozess zu kommen ist die Verwendung von amorphem Valsartan. Ein Verfahren nach dem reproduzierbar und sicher ein vollständig amorpher Wirkstoff hergestellt werden kann, ist allerdings bisher nicht bekannt. Nach den experimentellen Erfahrungen des Erfinders der vorliegenden Anmeldung treten beim Fällungsprozess der amorphen Form öfters auch heterogene Produkte, d. h. Mischungen mit kristallinen und amorphen Anteilen auf, was dazu führt, dass der Fällungsprozess wiederholt werden muss. Neben unvermeidbaren Substanzverlusten ist somit zusätzlicher Herstellungsaufwand erforderlich. Allerdings liegen bislang noch keine Erfahrungen zur Stabilität von amorpher Substanz vor, insbesondere auch hinsichtlich einer nicht auszuschließenden Phasenumwandlung im Verlaufe der Lagerung bzw. der weiteren Verarbeitung.

Darüber hinaus stellt die Herstellung von pharmazeutischen Zubereitungen mit amorphem Wirkstoff ein generelles Problem dar, zu dessen Lösung ein zum Teil erheblicher technischer Aufwand benötigt wird: der einfache Weg einer Direktverpressung von Pulvermischungen mit amorphem Wirkstoff, sofern bei dem im vorliegenden Fall von Valsartan erforderlichen hohen Wirkstoffgehalt eines Arzneimittels überhaupt möglich, beinhaltet das Risiko von Entmischungsvorgängen, die zu inhomogener, pharmazeutisch nicht akzeptabler Wirkstoffverteilung führen. Um einerseits Schwankungen des Wirkstoffgehaltes zu vermeiden und auf arzneibuchmäßig zulässige Grenzen zu beschränken, und andererseits verarbeitungsfähigen Wirkstoff zu erhalten, sind z. B. Trocken- oder Feuchtgranulationsverfahren in vorgeschalteten Arbeitsstufen erforderlich. Nach Trocknung, Zerkleinerung und Klassierung erhält man schließlich pressfähige Granulate. Wie aus WO 00/38676 bzw.
EP 1 140 071 B1 bekannt ist, muss selbst bei kristallinem Valsartan ein Trockengranulationsverfahren bei der Verarbeitung des Wirkstoffes zu Arzneiformen eingesetzt werden.

Entsprechende technisch und arbeitsmäßig aufwendige Verfahren sind dem Fachmann bekannt (z. B. Die Tablette, W. A. Ritschel und A. Bauer-Brandl., 2. Aufl., ECV-Edition Cantor Verlag, [2002]), und können das Problem im Falle von kristallinem Valsartan nur mit hohem arbeitstechnischen Aufwand lösen. Die bekannten Verfahren zur Herstellung von pharmazeutischer Formulierung mit amorphem Valsartan sind, sofern diese überhaupt durchführbar sind, technisch zumindest vergleichbar aufwendig, sowie zeit- und kostenintensiv.

Daher ist es Aufgabe der vorliegenden Erfindung, ein einfaches und kostengünstiges Verfahren zur Herstellung von Valsartan-Pulversystemen zu entwickeln, die direkt zur Herstellung von pharmazeutischen Formulierungen eingesetzt werden können, wobei dieses Verfahren jedoch nicht auf eine besonders bevorzugte Wirkstofmorphologie eingeschränkt ist, und welches die oben diskutierten Nachteile vermeidet.

Diese Aufgabe wird erfindungsgemäß durch ein Verfahren zur Herstellung von Valsartan-Adsorbaten und/oder von dessen Solvaten oder Hydraten gemäß Anspruch 1 gelöst. Dabei geht man von einer Lösung des Valsartans oder eines seiner pharmazeutisch akzeptablen Salze und/oder deren Solvate oder Hydrate in einem vorwiegend organischen Lösungsmittel aus, wobei der Wirkstoff im organischen Lösungsmittel gelöst vorliegt, suspendiert darin das Adsorbermaterial und entfernt das Lösungsmittel, was insbesondere durch Trocknen geschehen kann. Der Gesamtwassergehalt des Lösungsmittels beträgt erfindungsgemäß nicht mehr als 15 Vol.-%, bevorzugt nicht mehr als 5 Vol.-%. In einer bevorzugten Ausführungsform der Erfindung enthalten die Valsartan-Adsorbate den Wirkstoff oder eines seiner pharmazeutisch akzeptablen Salze und/oder deren Solvate oder Hydrate in fein verteilter amorpher Form. Das amorphe Valsartan oder eines seiner Salze kann sowohl in wasserfreier Form als auch in Form von Solvaten oder Hydraten vorliegen. Die Erfindung betrifft weiterhin nach dem Verfahren herstellbare Adsorbate des Valsartans und/oder pharmazeutisch akzeptable Salze des Valsartans und/oder entsprechende Solvate oder Hydrate davon.

Pharmazeutisch akzeptable Salze von Valsartan mit Basen sind typischerweise Basenadditionssalze mit Metallsalzen, wie z. B. Alkali- oder Erdalkalimetallsalzen, typischerweise Natrium-, Kalium-, Calcium- oder Magnesium-Salze oder Salze mit Ammoniak oder organischen Aminen, wie z. B. mit Morpholin, Thiomorpholin, Piperidin, Pyrrolidin, Mono-, Di- oder niederen Trialkylaminen, typischerweise Ethylamin, tert.-Butylamin, Diethylamin, Diisopropylamin, Dibutylamin, Triethylamin, Tributylamin oder Dimethylpropylamin. Es sind auch Additionssalze von Valsartan mit Mono-, Di- oder Trihydroxyniederalkylaminen, typischerweise Mono-, Di- oder Triethanolamin möglich. Entsprechende innere Salze können ebenfalls verwendet werden.

Die Erfindung betrifft weiterhin pharmazeutische Formulierungen, enthaltend diese neuen Valsartan-Adsorbate. Die pharmazeutischen Formulierungen enthalten gegebenenfalls weitere Hilfsstoffe und können in die gewünschte Darreichungsform überführt werden. Besonders bevorzugt sind den Wirkstoff schnell freisetzende, durch Direktverpressung hergestellte Tabletten.

Die erfindungsgemäßen pharmazeutischen Formulierungen, enthaltend Valsartan-Adsorbate als AT₁-Rezeptor-Antagonist, können zur Behandlung von Krankheiten eingesetzt werden, die sich beispielsweise wie folgt beschreiben lassen:
(a) Hypertonie, Herzinsuffizienz, Nierenversagen, insbesondere chronisches Nierenversagen, Restenose nach perkutaner transluminaler Angioplastie, und Restenose nach coronarer Arterienbypassoperation,
(b) Arteriosklerose, Insulinresistenz und Syndrom X, Diabetes Mellitus Typ 2, Fettleibigkeit, Nephropathie, Nierenversagen, z. B. chronisches Nierenversagen, Hypothyreose, Zustand nach Herzinfarkt, coronare Herzkrankheiten, Altershypertonie, familiäre dyslipidämische Hypertonie, alle vorgenannten Krankheiten verbunden mit oder ohne Hypertonie,
(c) Endotheliale Dysfunktion in Verbindung mit oder ohne Hypertonie,
(d) Hyperlipidämie, Hyperlipoproteinämie, Arteriosklerose und Hypercholesterinämie,
(e) Glaukom.

Für das erfindungsgemäße Verfahren zur Herstellung der Valsartan-Adsorbate eignen sich organische Lösungsmittel, die den pharmazeutischen Wirkstoff lösen. Die organischen Lösungsmittel sind insbesondere ausgewählt aus der Gruppe der niederen Alkanole mit 1 bis 4 Kohlenstoffatomen, der Gruppe der Ether, der Gruppe der Ester, der Gruppe der aliphatischen Ketone und der Gruppe der halogenierten Kohlenwasserstoffe, sowie Gemische der vorgenannten Lösungsmittel. Besonders bevorzugt sind Methanol, Ethanol, Isopropanol, n-Propanol, Aceton und andere Lösungsmittel wie Ethylacetat, Methylethylketon, Diisopropylether, MTBE (Methyltert.-butylether), Dichlormethan, Petrolether, Aceton, Hexan, ein Aceton/Wasser-Gemisch, ein Ethylacetat/Hexan-Gemisch, ein Dichlormethan/Ethylacetat-Gemisch, sowie weitere Gemische der vorgenannten Lösungsmittel.

Als Adsorbermaterialien werden erfindungsgemäß pharmazeutisch akzeptable Hilfsstoffe eingesetzt, die für eine schnelle Wirkstofffreisetzung geeignet sind, wie Cellulosen und Cellulosederivate, insbesondere Lactose, Maltodextrin, Stärken, Cyclodextrine, Polydextrosen oder Gemische der vorgenannten Substanzen. Mikrokristalline Cellulose, Lactose und Mannit sind erfmdungsgemäß bevorzugt. Zur Verbesserung von Fliesseigenschaften können Siliziumdioxid- oder Titiandioxid- haltige Zusätze erfolgen.

Das Verhältnis des pharmazeutischen Wirkstoffes zum Adsorbermaterial liegt erfindungsgemäß im Bereich von 1:0,1 bis 1:10, wobei insbesondere ein Bereich von 1:0,5 bis 1:2 bevorzugt wird.

Für die Herstellung der pharmazeutischen Formulierungen, wobei insbesondere Tabletten bevorzugt sind, können alle üblichen pharmazeutischen Hilfsstoffe verwendet werden. Als Füllstoffe können z. B. Cellulosen und Cellulosederivate (z.B. mikrokristalline Cellulose, native Cellulose, Hydroxypropylcellulose, Hydroxypropylmethylcellulose, Methylcellulose), Zucker (z.B. Lactose, Fructose, Saccharose, Glukose, Maltose), Zuckeralkohole (z. B. Lactit, Mannit, Sorbit, Xylit), anorganische Füllstoffe (z.B. Calciumphosphate und Calciumsulfate) und Stärken (z.B. Maisstärke, Kartoffelstärke, Weizenstärke, Dextrine, pregelatinisierte Stärken) verwendet werden. Darüber hinaus können alle weiteren Hilfsstoffe, die dem Fachmann aufgrund seines galenischen Basiswissens bekannt sind, wie z. B. Schmiermittel, Zerfallhilfsmittel, Netzmittel, Stoffe zur Verbesserung des Fliessverhaltens, sonstige Zusatzstoffe, Stabilisatoren sowie Aromen, Farbpigmente und Farbstoffe zur Herstellung der erfindungsgemäßen Arzneiformulierungen eingesetzt werden (entsprechende Informationen kann der Fachmann z. B. aus: Die Tablette, W. A. Ritschel und A. Bauer-Brandl., 2. Aufl., ECV-Edition Cantor Verlag, [2002] entnehmen).

Der Anteil an Bindemitteln in der Gesamtmischung der Arzneimittelzubereitung ist bevorzugt 0 bis 20% (m/m), der Anteil an Füll- und Hilfsstoffen in der Gesamtmischung beträgt 2 bis 80%, bevorzugt von 5 bis 25%.

Mit dem erfindungsgemäßen Verfahren werden überraschend stabile und homogene Adsorbate von Valsartan hergestellt, die keinerlei kristalline Wirkstoffanteile aufweisen. Diese Valsartan-Adsorbate werden als pharmazeutischer Wirkstoff in den erfindungsgemäßen Zubereitungen eingesetzt.

Im Rahmen dieser Erfindung können weiterhin die jeweiligen Hydrate, Solvate oder Salze des Valsartans eingesetzt werden, die insbesondere im Verlaufe der Wirkstoffherstellung am Ende des Syntheseweges in der Lösung anfallen können, was die Isolation des reinen Wirkstoffes vermeidet.

Es wurde nun erfindungsgemäß ein Verfahren gefunden, das ausgehend von einer Lösung des Valsartans in einem organischen Lösungsmittel zu direkt zur Arzneiform weiterverarbeitbaren Wirkstoff-Adsorbaten führt.

Die Valsartan-Wirkstofflösung kann grundsätzlich in einer Ausführungsform der Erfindung durch Auflösen des Wirkstoffs oder eines Salzes in einem geeigneten organischen Lösungsmittel hergestellt werden; vorteilhafter ist jedoch, eine im Rahmen der Synthese ohnehin anfallende Wirkstofflösung ohne Isolierung des Valsartans direkt zu verwenden.

Beispielsweise kann das Valsartan gemäß EP 0 443 983 B1 hergestellt werden, wobei dann auf Rekristallisierungsschritte, z. B. durch Lösen in Diisopropylether oder Ethylacetat, verzichtet wird. Statt dessen wird das Adsorbermaterial in der Wirkstofflösung suspendiert und das Lösungsmittel durch Trocknen später entfernt. Die Art des organischen Lösungsmittels ergibt sich dann im Einzelfall aus dem abschließenden Synthese-Schritt der gewählten Wirkstoffherstellung.

Zu dieser organischen Wirkstofflösung wird ein darin nicht bzw. gering löslicher pharmazeutisch akzeptabler Hilfsstoff als Adsorbermaterial zugegeben, gut benetzt und unmittelbar danach das Lösungsmittel durch Trocknen entfernt. Der Trocknungsvorgang kann durch Temperieren, Anlegen eines Vakuums z. B. Sublimationstrocknung oder auch durch Versprühen unterstützt werden. Er wird vorteilhaft so gestaltet, dass durch geeigneten mechanischen Einfluss (z. B. Dreh-, Taumel-, Rührbewegung) eine einheitliche Verteilung gegeben ist. Das Lösungsmittel kann durch Arbeiten im geschlossenen System zurückgewonnen und für den Folgeprozess wieder eingesetzt werden. Erfindungsgemäß ist, dass eine Ausfällung und Isolierung des Valsartans entfällt. Gemäß dem beschriebenen Verfahren hergestellte Adsorbate können direkt zur Weiterverarbeitung zu Arzneiformen wie Tabletten, Kapseln, Pellets oder Granulaten eingesetzt werden, bevorzugt zur Weiterverarbeitung mittels eines Direktkompressionsverfahrens.

Optional können die so erhaltenen Adsorbate oder Arzneiformen für spezielle Anwendungen weiter mit Überzügen aus pharmazeutischen Polymethacrylaten, wie z. B. Eudragit® - Filme, Methylcellulosen, Ethylcellulosen, Hydroxypropylmethylcellulosen, Celluloseacetatphthalaten und/oder Schellack versehen werden, um einen bestimmten Anwendungszweck, z. B. kontrollierte Wirkstoff-Freisetzung und/oder Geschmacksabdeckung zu erzielen. Hierfür stehen dem pharmazeutischen Fachmann ausreichende technische Möglichkeiten zur Verfügung.

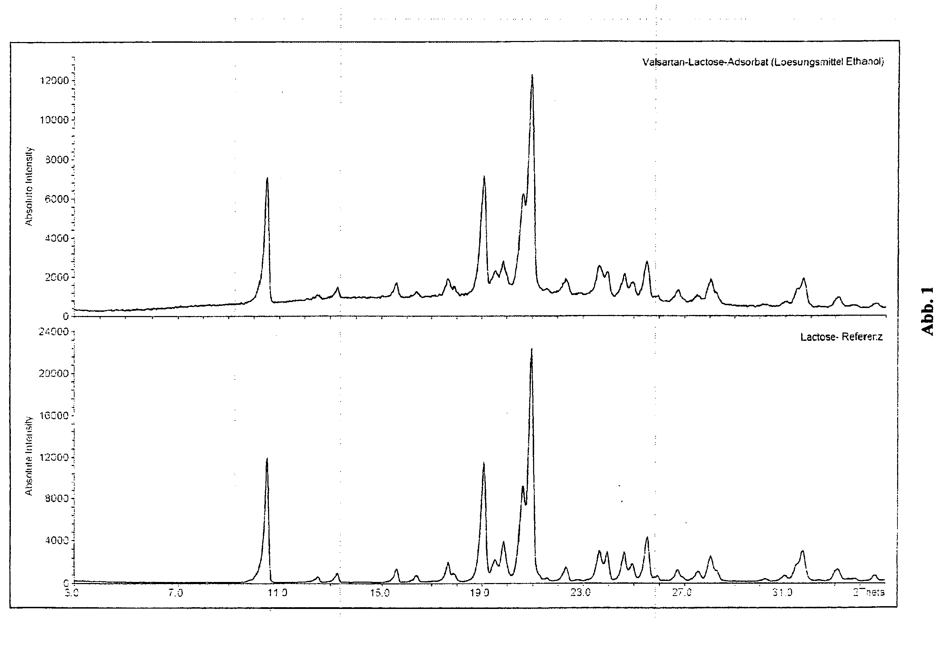

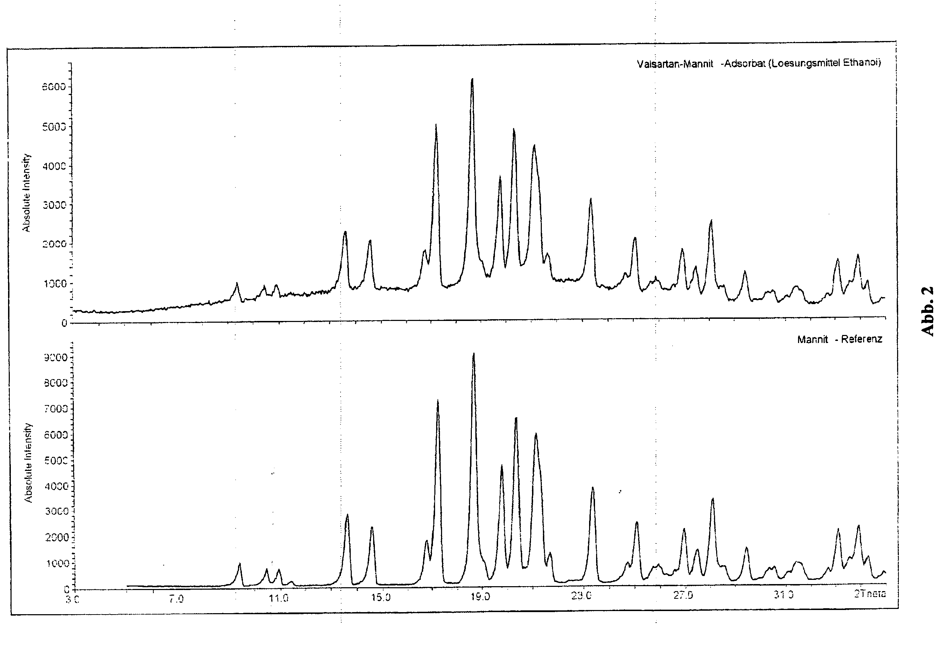

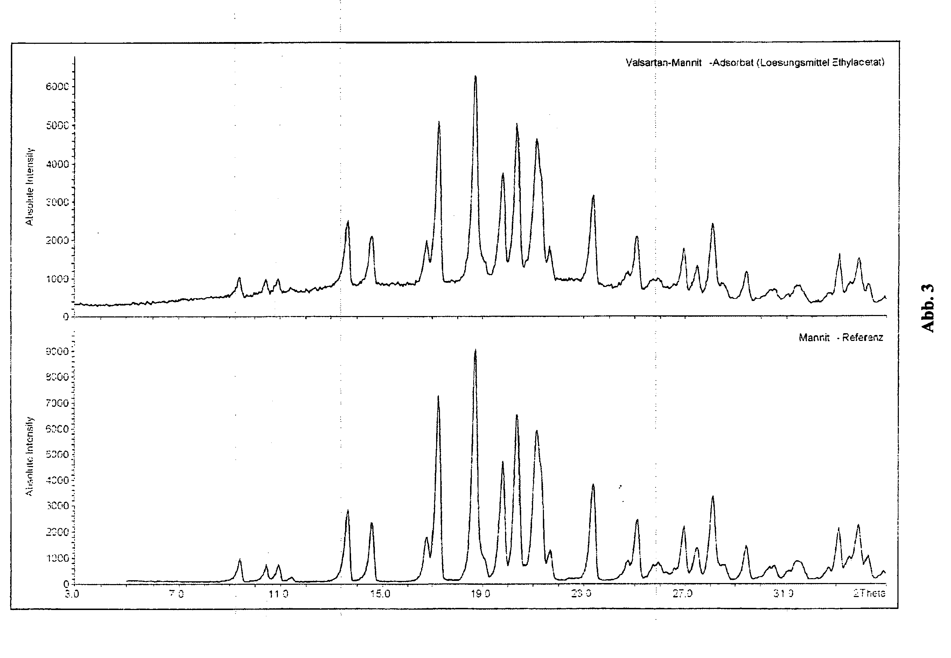

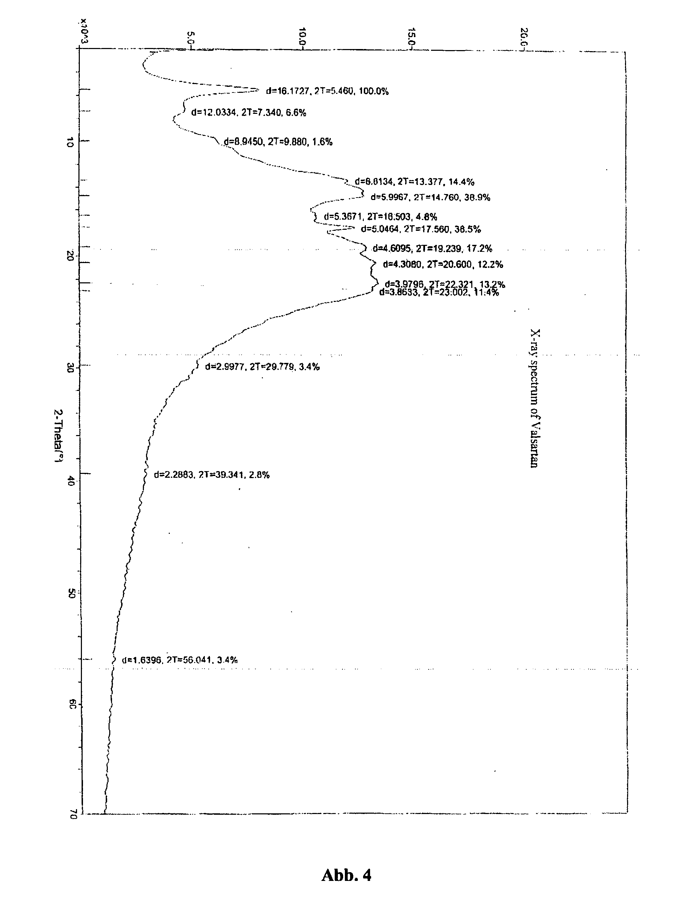

Überraschend wurde gefunden, dass nach dem erfindungsgemäßen Verfahren hergestellte Adsorbate den Wirkstoff in der für Arzneimittel erforderlichen homogenen Verteilung aufweisen, und diesen uneingeschränkt freisetzen. Die nach diesem Verfahren hergestellten Adsorbate binden den Wirkstoff derart, dass es nicht zur Ausbildung von wirkstofftypischen Kristallsttukturen kommt. Dies konnte anhand von röntgendiffraktometrischen Messungen gezeigt werden (siehe Abbildungen 1 bis 3). Zum Vergleich zeigt Abbildung 4 ein Pulverröntgenbeugungsdiagramm mit substanztypischer Kristallstruktur.

Die genannten Eigenschaften bleiben auch insbesondere dann erhalten, wenn die Valsartan-Adsorbate zu Arzneiformen, wie z. B. Tabletten verarbeitet werden. Darüber hinaus führt diese direkte Verarbeitung zu keiner Veränderung der Morphologie oder des Gehalts an Neben- und Abbauprodukten ( = Summe aller Verunreinigungen) auf dem Wege vom Wirkstoff zur Arzneiform (Tablette).

Die Erfindung wird nun anhand von Beispielen und Figuren näher erläutert.

### Beispiele 1 bis 3

### Verwendete Analysemethoden

*1. HPLC-Methode zur Bestimmung des Wirkstoffgehaltes bzw. der Summe aller Verunreinigungen gemäß USP 27 - In Process Revision - Pharmacopoeial Forum, Vol. 29 [Nov.-Dec. 2003]*
*2. Wirkstoff-Freisetzung (Dissolutionstest) gemäß USP 27 - In Process Revision - Pharmacopoeial Forum, Vol. 29 [Nov.-Dec. 2003] (Soll: mind. 80% Freisetzung nach 30 Min.)*
*3. Die Pulverröntgenbeugungsdiagramme wurden wie folgt gemessen:*

| | |
|---|---|
| Gerät: | STADI P Transmissions-Diffraktometer Cu-Ka₁-Strahlung (1 = 1.54056Å), U = 40 kV, I = 30 mA Sekundärstrahl-Monochromator (eben, Graphit) Detektor: Scintillationszähler |
| | |
| Spaltbreite: | 2 x 8 mm; 0,7 mm; 0,35 mm |
| | |
| Linear PSD: | 2θ = 2° bis 35°, 5s / 0,04° stufenweise |
| | |
| Probe: | Pulver, Reflektionsmodus |

### Abbildungen 1 bis 3

- *Abb. 1:*: *Pulverröntgenbeugungsdiagramme eines erfindungsgemäßen Valsartan-Lactose-Adsorbates aus Ethanol (im Verhältnis 1:1) in der oberen Kurve, sowie als Vergleich in der unteren Kurve Lactose alleine*
- *Abb. 2:*: *Pulverröntgenbeugungsdiagramme eines erfindungsgemäßen Valsartan-Mannit-Adsorbates aus Ethanol (im Verhältnis 1:1) in der oberen Kurve, sowie als Vergleich in der unteren Kurve Mannit alleine*
- *Abb. 3:*: *Pulverröntgenbeugungsdiagramme eines erfindungsgemäßen Valsartan-Mannit-Adsorbates aus Ethylacetat (im Verhältnis 1:1) in der oberen Kurve, sowie als Vergleich in der unteren Kurve Mannit alleine*
- *Abb. 4*: *Pulverröntgenbeugungsdiagramme von Valsartan (kristallisiert aus Diisopropylether), zeigend eine heterogene Mischung von kristallinen und amorphen Phasen*

### Beispiel 1: Valsartan-Lactose-Adsorbat

Zu einer Lösung von heterogenem Valsartan (0,05 g/ml) in Ethanol werden 0,05 g/ml Lactose (Lactopress®, wasserfrei) zugegeben und gleichmäßig suspendiert. Das Lösungsmittel wird nun unter permanenter Bewegung und Vakuum (Rotationsverdampfer) bei 25°C abgetrocknet. Die Mischung wird abschließend zur Entfernung von Restlösemittel kurzzeitig bei 35°C nachgetrocknet.

| | |
|---|---|
| Theoretischer Wirkstoffgehalt des Adsorbats | 50% |
| Röntgenbeugungsdiagramm | siehe Abbildung 1 |

| **Wirkstoffgehalt mittels HPLC** | **Adsorbat (% Valsartan)** | **175 mg Tablette (mg Valsartan)** |
|---|---|---|
| Probe Nr. 1 | 50,1 | 80,1 |
| Probe Nr. 2 | 50,2 | 80,0 |

Aus dem Adsorbat wurden Valsartan-Tabletten (Endgewicht ca. 175 mg) mittels Direktverpressung nach folgender Zusammensetzung hergestellt:
■ Valsartan-Lactose-Adsorbat, entsprechend 80 mg Valsartan 160 mg
■ Hilfsstoffe (Croscarmellose-Natrium, Natriumlaurylsulfat, Siliziumdioxid, Magnesiumstearat) in den üblichen Mengen 15 mg

Die verwendeten Mengen der weiteren Hilfsstoffe sind dem Fachmann aufgrund seines Basiswissens bekannt und können Standardwerken zur Formulierung von Tabletten, wie zum Beispiel Ritschel et al., Die Tablette, Editio Cantor - Aulendorf, 2. Auflage [2002] entnommen werden.

Eigenschaften der pressfertigen Mischung und der Tabletten:
■ Kompressibilität und Fließfähigkeit: befriedigend bis gut
■ Mittlere Härte: 92 N
■ Abrieb: 0,2% (bestimmt nach Ph. Eur.)
■ Freisetzung: 90% nach 30 Min.
■ Gehalt: siehe Tabelle
■ Summe aller Verunreinigungen: Granulat: 0,08%; Tablette: 0,08%
Die so erhaltenen Tabletten können mit einem Überzug versehen werden.

### Beispiel 2: Valsartan-Mannit-Adsorbat

Zu einer Lösung von heterogenem Valsartan (0,05 g/ml) in Ethanol werden 0,05 g/ml Mannit (Mannogem®) zugegeben und gleichmäßig suspendiert. Das Lösungsmittel wird nun unter permanenter Bewegung und Vakuum (Rotationsverdampfer) bei 25°C abgetrocknet. Die Mischung wird abschließend zur Entfernung von Restlösemittel kurzzeitig bei 35°C nachgetrocknet.

| | |
|---|---|
| Theoretischer Wirkstoffgehalt des Adsorbats | 50% |
| Röntgenbeugungsdiagramm | siehe Abbildung 2 |

| **Wirkstoffgehalt mittels HPLC** | **Adsorbat (% Valsartan)** | **175 mg Tablette (mg Valsartan)** |
|---|---|---|
| Probe Nr. 1 | 50,1 | 79,9 |
| Probe Nr. 2 | 49,9 | 80,2 |

Aus dem Adsorbat wurden Valsartan-Tabletten (Endgewicht ca. 175 mg) mittels Direktverpressung nach folgender Zusammensetzung hergestellt:
■ Valsartan-Mannit-Adsorbat, entsprechend 80 mg Valsartan 160 mg
■ Hilfsstoffe (wie in Beispiel 1) 15 mg

Eigenschaften der pressfertigen Mischung und der Tabletten:
■ Kompressibilität und Fließfähigkeit: befriedigend bis gut
■ Mittlere Härte: 93 N
■ Abrieb: 0,2% (bestimmt nach Ph. Eur.)
■ Freisetzung: 96% nach 30 Min.
■ Gehalt: siehe Tabelle
■ Summe aller Verunreinigungen: Granulat: 0,07%; Tablette: 0,07%

Die so erhaltenen Tabletten können mit einem Überzug versehen werden.

### Beispiel 3: Valsartan-Mannit-Adsorbat

Zu einer Lösung von heterogenem Valsartan (0,05 g/ml) in Ethylacetat werden 0,05 g/ml Mannit (Mannogem®) zugegeben und gleichmäßig suspendiert. Das Lösungsmittel wird nun unter permanenter Bewegung und Vakuum (Rotationsverdampfer) bei 25°C abgetrocknet. Die Mischung wird abschließend zur Entfernung von Restlösemittel kurzzeitig bei 35°C nachgetrocknet.

| | |
|---|---|
| Theoretischer Wirkstoffgehalt des Adsorbats | 50% |
| Röntgenbeugungsdiagramm | siehe Abbildung 3 |

| **Wirkstoffgehalt mittels HPLC** | **Adsorbat (% Valsartan)** | **350 mg Tablette (mg Valsartan)** |
|---|---|---|
| Probe Nr. 1 | 49,8 | 161,4 |
| Probe Nr. 2 | 51,0 | 160,9 |

Aus dem Adsorbat wurden Valsartan-Tabletten (Endgewicht ca. 350 mg) mittels Direktverpressung nach folgender Zusammensetzung hergestellt:
■ Valsartan-Mannit-Adsorbat, entsprechend 160 mg Valsartan 320 mg
■ Hilfsstoffe (wie in Beispiel 1) 30 mg

Eigenschaften der pressfertigen Mischung und der Tabletten:
■ Kompressibilität und Fließfähigkeit: befriedigend bis gut
■ Mittlere Härte: 112 N
■ Abrieb: 0,2% (bestimmt nach Ph. Eur.)
■ Freisetzung: 91% nach 30 Min.
■ Gehalt: siehe Tabelle
■ Summe aller Verunreinigungen: Granulat: 0,04%; Tablette: 0,04%

Die so erhaltenen Tabletten können mit einem Überzug versehen werden.

## Patentansprüche

1. Verfahren zur Herstellung von Valsartan-Adsorbaten und/oder von dessen Solvaten oder Hydraten, **dadurch gekennzeichnet, dass** man von einer Lösung des Valsartans oder eines seiner pharmazeutisch akzeptablen Salze und/oder deren Solvate oder Hydrate in wenigstens einem organischen Lösungsmittel, mit einem Gesamtwassergehalt des Lösungsmittels von nicht mehr als 15 Vol.-%, bevorzugt nicht mehr als 5 Vol.-%, ausgeht, Adsorbermaterial, ausgewählt aus der Gruppe, bestehend aus Cellulosen, Cellulosederivaten, Polyolen, Zuckern, Zuckerderivaten, Maltodextrinen, Cyclodextrinen, Stärken, Polydextrosen oder Mischungen daraus, darin suspendiert und das Lösungsmittel entfernt.

2. Verfahren gemäß Anspruch 1, **dadurch gekennzeichnet, dass** die Adsorbate den Wirkstoff oder eines pharmazeutisch akzeptablen Salzes davon oder dessen Solvate oder Hydrate in fein verteilter amorpher Form enthalten.

3. Verfahren gemäß irgendeinem der Ansprüche 1 oder 2, **dadurch gekennzeichnet, dass** man ein Verhältnis von Wirkstoff zu Adsorbermaterial im Bereich von 1:0,1 bis 1:10, insbesondere im Bereich von 1:0,5 bis 1:2 einstellt.

4. Verfahren gemäß einem oder mehreren der vorhergehenden Ansprüche 1 bis 3, **dadurch gekennzeichnet, dass** man als Lösungsmittel organische Lösungsmittel mit einem Gesamtwasseranteil von nicht mehr als 15 Vol.%, bevorzugt nicht mehr als 5 Vol.%, alleine oder in Mischungen einsetzt, wobei die organischen Lösungsmittel ausgewählt sind aus der Gruppe der niederen Alkanole mit 1 bis 4 Kohlenstoffatomen, der Gruppe der Ether, der Gruppe der Ester, der Gruppe der aliphatischen Ketone und der Gruppe der halogenierten Kohlenwasserstoffe und deren Gemischen.

5. Verfahren gemäß Anspruch 4, **dadurch gekennzeichnet, dass** man als Lösungsmittel ein Lösungsmittel aus der Gruppe, bestehend aus Methanol, Ethanol, Isopropanol, n-Propanol, Aceton, Etylacetat, Methylethylketon, Methyl-t-butylether, Dichlormethan, Petrolether, Hexan, einem Aceton/Wasser-Gemisch, einem Ethylacetat/Hexan-Gemisch, einem Gemisch aus Dichlormethan und Ethylacetat sowie weiteren Gemischen der vorgenannten Lösungsmittel, einsetzt.

6. Verfahren gemäß einem der Ansprüche 1 bis 5, **dadurch gekennzeichnet, dass** man eine Wirkstofflösung einsetzt, die im Rahmen der Valsartansynthese anfällt.

7. Valsartan-Adsorbate und dessen Solvate, **dadurch gekennzeichnet, dass** sie nach dem Verfahren gemäß einem oder mehreren der Ansprüche 1 bis 6 herstellbar sind.

8. Pharmazeutische Formulierung mit mindestens einem aktiven Wirkstoff und gegebenenfalls weiteren pharmazeutisch akzeptablen Hilfsstoffen, **dadurch gekennzeichnet, dass** sie als Wirkstoff ein Valsartan-Adsorbat gemäß einem oder mehreren der vorhergehenden Ansprüche 1 bis 7 enthält.

9. Pharmazeutische Formulierung gemäß Anspruch 8, **dadurch gekennzeichnet, dass** sie in Form von Tabletten, Kapseln, Pellets und Granulaten vorliegt, die auf bekannte Weise mit üblichen pharmazeutisch akzeptablen Hilfsstoffen herstellbar ist.

10. Pharmazeutische Formulierung gemäß Anspruch 8 oder 9 in Form von den Wirkstoff schnell freisetzenden, durch Direktverpressung hergestellten Tabletten.

11. Pharmazeutische Formulierung gemäß einem der Ansprüche 8 bis 10, **dadurch gekennzeichnet, dass** der Anteil an Bindemittel in der Gesamtmischung der Arzneimittelzubereitung von 0 bis zu 20 % (m/m) beträgt.

12. Pharmazeutische Zubereitung gemäß irgendeinem der Ansprüche 8 bis 10, **dadurch gekennzeichnet, dass** der Anteil an Füll- und Hilfsstoffen in der Gesamtmischung der Arzneimittelzubereitung 2 bis 80 Gew.-%, bevorzugt 5 bis 25 Gew.-%, beträgt.

## Claims

1. Method of producing valsartan adsorbates and/or solvates or hydrates thereof, **characterised in that** one starts from a solution of valsartan or of one of its pharmaceutically acceptable salts and/or solvates or hydrates thereof in at least one organic solvent, having a total water content of the solvent of not more than 15 % by vol., preferably not more than 5 % by vol., one suspends therein adsorbent material, selected from the group comprising celluloses, cellulose derivatives, polyols, sugars, sugar derivatives, maltodextrins, cyclodextrins, starches, polydextroses or mixtures thereof, and one removes the solvent.

2. Method according to claim 1, **characterised in that** the adsorbates contain the active substance or a pharmaceutically acceptable salt thereof or solvates or hydrates thereof in finely distributed, amorphous form.

3. Method according to any of claims 1 or 2, **characterised in that** a ratio of active substance to adsorbent material is set in the range of 1:0.1 to 1:10, more especially in the range of 1:0.5 to 1:2.

4. Method according to one or more of the preceding claims 1 to 3, **characterised in that** organic solvents, having a total water proportion of not more than 15 % by vol., preferably not more than 5 % by vol., alone or in mixtures, are used as the solvent, the organic solvents being selected from the group of low alkanols having 1 to 4 carbon atoms, the group of ethers, the group of esters, the group of aliphatic ketones and the group of halogenated hydrocarbons and mixtures thereof.

5. Method according to claim 4, **characterised in that**, used as the solvent, is a solvent from the group comprising methanol, ethanol, isopropanol, n-propanol, acetone, ethyl acetate, methyl ethyl ketone, methyl-t-butyl ether, dichloromethane, petrol ether, hexane, an acetone/water mixture, an ethyl acetate/hexane mixture, a mixture formed from dichloromethane and ethyl acetate as well as additional mixtures of the aforementioned solvents.

6. Method according to one of claims 1 to 5, **characterised in that** an active substance solution is used which is obtained within the scope of valsartan synthesis.

7. Valsartan adsorbates and solvates thereof, **characterised in that** they are producible by the method according to one or more of claims 1 to 6.

8. Pharmaceutical formulation having at least one active substance and possibly additional pharmaceutically acceptable adjuvants, **characterised in that** it contains, as the active substance, a valsartan adsorbate according to one or more of the preceding claims 1 to 7.

9. Pharmaceutical formulation according to claim 8, **characterised in that** it is in the form of tablets, capsules, pellets and granules and is producible in known manner with conventional pharmaceutically acceptable adjuvants.

10. Pharmaceutical formulation according to claim 8 or 9 in the form of tablets which release the active substance rapidly and are produced by direct press-moulding.

11. Pharmaceutical formulation according to one of claims 8 to 10, **characterised in that** the proportion of bonding agent in the total mixture of the drug preparation amounts to between 0 and 20 % (m/m).

12. Pharmaceutical preparation according to any of claims 8 to 10, **characterised in that** the proportion of filler substances and adjuvants in the total mixture of the drug preparation amounts to between 2 and 80 % by wt., preferably to between 5 and 25 % by wt.

## Revendications

1. Procédé de préparation d'adsorbats de Valsartan et/ou de solvates ou d'hydrates, **caractérisé en ce que** l'on part d'une solution du Valsartan ou d'un de ses sels et/ou de leurs solvates ou de leurs hydrates pharmaceutiquement acceptables dans au moins un solvant organique, avec une teneur en eau totale du solvant de pas plus de 15 % en volume, de préférence de pas plus de 5 % en volume, que l'on y met en suspension un matériau adsorbant, choisi parmi le groupe qui consiste en les celluloses, les dérivés de la cellulose, les polyols, les sucres, les dérivés de sucres, les maltodextrines, les cyclodextrines, les amidons, les polydextroses ou des mélanges de ces derniers, et que l'on élimine le solvant.

2. Procédé selon la revendication 1, **caractérisé en ce que** les adsorbats contiennent le principe actif ou un sel pharmaceutiquement acceptable de celui-ci ou de ses solvates ou de ses hydrates sous forme amorphe finement dispersée.

3. Procédé selon l'une quelconque des revendications 1 ou 2, **caractérisé en ce que** l'on ajuste un rapport du principe actif au matériau adsorbant dans l'intervalle de 1 : 0,1 à 1 : 10, en particulier dans l'intervalle de 1 : 0,5 à 1 : 2.

4. Procédé selon l'une ou plusieurs des revendications précédentes 1 à 3, **caractérisé en ce que** l'on utilise comme solvants des solvants organiques avec une teneur totale en eau de pas plus de 15 % en volume, de préférence de pas plus de 5 % en volume, seuls ou en mélanges, sachant que les solvants organiques sont choisis parmi le groupe des alcanols de bas poids moléculaire avec 1 à 4 atomes de carbone, le groupe des éthers, le groupe des esters, le groupe des cétones aliphatiques et le groupe des hydrocarbures halogénés et de leurs mélanges.

5. Procédé selon la revendication 4, **caractérisé en ce que** l'on utilise comme solvant un solvant du groupe qui consiste en le méthanol, l'éthanol, l'isopropanol, le n-propanol, l'acétone, l'acétate d'éthyle, la méthyléthylcétone, l'éther de méthyle et de t-butyle, le dichlorométhane, l'éther de pétrole, l'hexane, un mélange d'eau et d'acétone, un mélange d'acétate d'éthyle et d'hexane, un mélange de dichlorométhane et d'acétate d'éthyle, ainsi que d'autres mélanges des solvants susmentionnés.

6. Procédé selon l'une des revendications 1 à 5, **caractérisé en ce que** l'on utilise une solution de principe actif qui est obtenue dans le cadre de la synthèse du Valsartan.

7. Adsorbats de Valsartan et ses solvates, **caractérisés en ce que** qu'ils peuvent être préparés d'après le procédé selon une ou plusieurs des revendications 1 à 6.

8. Formulation pharmaceutique avec au moins un principe actif et, le cas échéant, d'autres substances auxiliaires pharmaceutiquement acceptables, **caractérisée en ce qu'**elle contient comme principe actif un adsorbat de Valsartan selon une ou plusieurs des revendications 1 à 7.

9. Formulation pharmaceutique selon la revendication 8, **caractérisée en ce qu'**elle se présente sous forme de comprimés, de capsules, de pellets ou de granulés qui peuvent être préparés de la façon connue avec des substances auxiliaires habituelles, pharmaceutiquement acceptables.

10. Formulation pharmaceutique selon la revendication 8 ou 9 sous forme de comprimés libérant le principe actif rapidement, préparés par compression directe.

11. Formulation pharmaceutique selon l'une des revendications 8 à 10, **caractérisée en ce que** la proportion de liant dans l'ensemble du mélange de la préparation du médicament est de 0 à 20 % (m/m).

12. Formulation pharmaceutique selon l'une quelconque des revendications 8 à 10, **caractérisée en ce que** la proportion de charges et de substances auxiliaires dans l'ensemble du mélange de la préparation du médicament est de 2 à 80 % en poids, de préférence de 5 à 25 % en poids.
